(19) Europäisches Patentamt
European Patent Office
Office européen des brevets

(11) **EP 4 181 149 A1**

(12) **EUROPEAN PATENT APPLICATION**

(43) Date of publication:
**17.05.2023 Bulletin 2023/20**

(21) Application number: **21216788.6**

(22) Date of filing: **22.12.2021**

(51) International Patent Classification (IPC):
**G16H 20/17** (2018.01)

(52) Cooperative Patent Classification (CPC):
**G16H 20/17**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.11.2021 US 202163277797 P**

(71) Applicant: **Koninklijke Philips N.V.**
**5656 AG Eindhoven (NL)**

(72) Inventors:
• **RAHMAN, Asif**
  **Eindhoven (NL)**
• **CHANG, Yale**
  **Eindhoven (NL)**
• **GHOSH, Erina**
  **Eindhoven (NL)**

(74) Representative: **Philips Intellectual Property & Standards**
**High Tech Campus 52**
**5656 AG Eindhoven (NL)**

(54) **CONTROL OF INTRAVENOUS FLUID APPLICATION**

(57) The invention relates to a system (200) for use in intravenous fluid application to a patient. The system uses a fluid balance control model trained to determine a target fluid balance based on a state of the patient. A state of the patient, including obtaining current measurements of one or more physiological parameters, is obtained and fed to the fluid balance control model to determine the target fluid balance, which is then output.

Fig. 4

**Description**

FIELD OF THE INVENTION

The invention relates to a system for use in intravenous fluid application to a patient, to a computer-implemented method for use in intravenous fluid application. The invention further relates to a computer-readable medium.

BACKGROUND OF THE INVENTION

Intravenous therapy is a medical technique that delivers fluids, medications, and nutrition directly into a person's vein. Fluid management is the problem to decide on fluid application parameters, such as an amount of fluid to be administered, and its composition, for example whether to include medication in the fluid and how much. Fluid management in hospitalized patients typically depends on many different factors, including patient demographics, comorbidities, physiological state, and past interventions. Intravenous fluid therapy is dynamic and can change in short time spans as physicians challenge patients with fluid administration and measure the response.

Fluid management typically follows an assessment stage, a resuscitation stage, and a de-resuscitation stage. During the assessment stage, fluid management may involve assessing the hemodynamic instability of the patient to decide whether the patient need fluids. During the resuscitation and de-resuscitation phases, fluid management may involve deciding how much fluid to apply or remove. The resuscitation phase may aim at an overall net positive amount of fluids to be applied, that is, an overall positive fluid balance. The de-resuscitation phase may aim at an overall positive amount of fluids to be removed, that is, an overall negative fluid balance. De-resuscitation can for example involve diuretics and/or renal replacement therapy with net ultrafiltration to achieve active fluid removal. The de-resuscitation phase is typically characterized by discontinuation of invasive therapies and a transition to a negative fluid balance.

Fluid management is currently typically a manual process in which decisions are made by the clinician. The fluid management strategy that is selected, can in practice be different for each patient or situation, with clinicians typically using their personal experience to select the best strategy, as opposed to following strict protocols.

SUMMARY OF THE INVENTION

It would be desirable to provide systems and methods that allow previous experiences to be taken into account in intravenous fluid application in an automated way.

In accordance with an aspect of the invention, a system is provided, as defined by the claims. The system is for use in intravenous fluid application, namely, the system may be used to determine and output a target fluid balance that can be used to manage the intravenous fluid application: for example, in a fully automatic way, or as a recommendation that can be confirmed or otherwise taken into account by a clinician. In accordance with a further aspect, a computer-implemented method for use in intravenous fluid application to a patient is provided, as defined by the claims.

In order to determine the target fluid balance, the system may use a trained fluid balance control model. The fluid balance control model may be configured to determine a target fluid balance based on a state of a patient, for example, a recommendation to decrease the fluid balance by a range of -750 to -250 mL over the next four hours. The state may comprise measurements of one or more physiological parameters of the patient. In order to apply the model, the state of the patient may be obtained, which may involve obtaining current measurements of the one or more physiological parameters via a sensor interface. The fluid balance control model can then be applied to the state of the patient to determine the target fluid balance.

By applying the fluid balance control model to current measurements, the provided system can provide real-time recommendations on fluid dosage for the prescription of intravenous fluids, thus addressing the dynamic fluid administration problem. The use of a trained model, in other words an artificial intelligence system, allows this recommendation to be based on data about previous cases in which intravenous fluid application was applied. In particular, the data on which the model was trained may include data about (short-term and/or long-term) clinical outcomes resulting from these previous fluid application instances. By using artificial intelligence, fluid management decisions can be objectivized and improved with respect to manual decisions. Moreover, by using clinical outcome data, these decisions can be guided by factors that the clinicians themselves may not typically take into account. It also becomes possible to steer for particular clinical outcomes or combinations of clinical outcomes.

The system may be for use in the de-resuscitation phase of the fluid therapy. Thus, the fluid balance control data may also be trained on data from the de-resuscitation phase of past fluid application instances, and the target fluid balance recommended by the model may be suitable for use in the de-resuscitation phase of the intravenous fluid application. The de-resuscitation phase is particularly relevant because there is a relatively large amount of leeway for the clinician to decide on the fluid application strategy in this phase, and decisions made in this phase can have a relatively large impact on eventual clinical outcomes. At the same time, it is not very well known in the state of the art how to steer for particular outcomes, making the use of the provided techniques particularly relevant. It is also possible to apply the provided techniques in other phases of the fluid therapy, for example, respective fluid balance control models may be used to obtain recommendations for use in the respective phases of the fluid therapy.

Specifically, the target fluid balance output by the control model may represent a recommended value, or a recommended range of values, for the fluid balance for a following fixed time period. In other words, the target fluid balance may indicate a target dosage level range for a following time period. The fluid balance may be indicated as a measure, $\Delta$FluidBalance, relative to a current fluid balance. For example, consecutive ranges of a given size may be used, e.g., intervals of size 500 mL, e.g., (-1250, 750), (-750, -250) up to (750, 1250). The fixed time period can be four hours, for example.

Optionally, the fluid balance control model may have been previously trained on a training dataset of past fluid application instances. Such a past fluid application instance may comprise a past state of a patient, a past target fluid balance, and a reward value indicating a clinical outcome resulting from applying the past target fluid balance to the patient in the past state. In reinforcement learning terms, the past fluid application instance may be considered to be a state/action/reward tuple. The past fluid application instance may additionally include a state corresponding to the reward value, e.g., a state resulting from applying the past target fluid balance to the patient in the past state. The model may be trained to select an action that optimizes the reward, e.g., the immediate reward or a cumulative reward over time, e.g., a finite- or infinite-horizon reward. For example, the state, action and reward may correspond to consecutive time blocks, e.g., of four hours. The past state may represent the condition of the patient over the first time block. The action may represent the fluid balance applied over the second time block. The reward may represent the condition of the patient over the third time block. Thus, the control model may use data collected during the first 4-hour state bloc to recommend a target fluid balance during the second 4-hour block that optimizes a reward in the third 4-hour block.

Interestingly, the reward typically incorporates short-term reward data coming from the time block following the taking of the action. This short-term reward data may vary throughout a fluid application. In particular, training data for training the model may include multiple state/action/reward, or current state/next state/action reward, tuples from a single fluid application to a patient. These different tuples can have different values for the reward. As a consequence, the model may provide recommendations that do not just aim at optimizing an eventual long-term clinical outcome, but can provide dynamic recommendations optimized also for, or even solely for, short-term outcomes.

Optionally, the clinical outcome indicated by the reward value may indicate how well an organ system is functioning. For example, the reward value may indicate one or more of a lactate level, a hemodynamic stability, a renal function, and a respiratory function. These are in practice important indicators that can be steered for by fluid application. By using such reward values, the fluid therapy recommendations can be selected to optimize for the functioning of the organ system, or, by combining the functioning of multiple organ systems, to optimize the overall function of the multiple organ systems combined.

Optionally, the clinical outcome may incorporate a subgroup mortality rate. The subgroup mortality rate may be determined by: assigning the patient to a subgroup based on a state of the patient resulting from applying the past target fluid balance, and obtaining a mortality rate of the assigned subgroup. Thus, effectively, the subgroup mortality rate provides an indicator of the overall health of patient shortly after the target fluid balance was applied, as indicated by the state of the patient. In particular, the subgroup mortality rate for the same patient during the same intravenous fluid application can vary over time. In other words, in contrast to using an eventual mortality of the patient itself, e.g., steering the fluid application towards long-term goals of the patient, a short-term steering to optimize the health during or shortly after the intravenous fluid application may be achieved. Because the relation between eventual mortality and the state of the patient at different points in the fluid application is relatively indirect, instead using a subgroup mortality rate allows mortality data to be used more effectively to steer for outcomes of the fluid application.

Optionally, the state of the patient may comprise: demographic information, one or more comorbidity indicators, one or more past intervention indicators, one or more further physiological parameters (e.g., one or more vital sign measurements and/or one or more lab result indicators), one or more medication indicators, or any combination of the above. These factors are found to be potentially relevant for fluid management. Interestingly, when fluid management is performed manually, these different types of information are typically not consulted throughout the fluid management process, e.g., some of this information may be consulted initially by the clinician but is typically not further taken into account regularly. By using a trainable model that takes these factors into account, they can be used for each target fluid balance that is derived.

Optionally, the fluid balance control model may have been trained using the reward value by reweighting the past fluid application instance based on the reward value. This way, the problem to select an optimal action given the current state may be dealt with as a cost-sensitive classification problem. The model may be trained to reproduce previously performed actions of a training dataset given previously recorded states. The reweighting may be used to encourage the model to output actions with higher rewards, by taking actions with higher reward into account more heavily in the training. In particular, samples may be reweighted such that the weight assigned to the past fluid therapy instance increases with its reward value and/or decreases with the likelihood that its action is be selected by a clinician, for example according to an inverse propensity scoring. Reweighting enables to optimize for rewards in a relatively simple way that is moreover compatible with a wide range of control models.

Optionally, the fluid balance control model may have been previously trained to maximize a cumulative reward defined based on the reward value, for example, according to a finite time horizon extending multiple states into the future, or according to an infinite time horizon. Thus, effectively, the problem to recommend an action from a state may be regarded as a reinforcement learning problem. To address this problem, various techniques known per se from reinforcement learning may be used. For example, the fluid balance control model may comprise a Q-function, also known as a quality function, learned using Q-learning, for example, by fitted Q-iteration or similar techniques. Applying the fluid balance control model may comprise evaluating the Q-function on the current state and respective target fluid balances to obtain respective Q-function values, and selecting the target fluid balance based on the respective Q-function values. By using cumulative rewards, possibly in combination with reinforcement learning, the recommendations provided by the fluid balance control model can better take into account effects of the fluid application at a longer time scale than just the direct short-term result of performing fluid application according to the recommended target fluid balance, for example, mid-term and/or long-term effects can bet taken into account.

Optionally, a fluid balance control model may have been previously trained on a labelled training dataset of patient states and associated target fluid balances that were previously selected by physicians. Thus, the fluid balance control model may be trained to reproduce the physician-selected target fluid balances. This control model may effectively represent what a physician would do in the situation and, especially in combination with other models that use reward values, e.g., based on reinforcement learning and/or inverse propensity scoring, can serve as a useful baseline to which a user can compare the recommendation by these other models.

Optionally, the target fluid balance may be used to provide fluid application parameters to an infusion pump. The fluid application parameters may indicate an amount of fluid and/or medication to be administered to the patient. The fluid application parameters may be determined automatically from the determined target fluid balance by a controller. For example, the controller may periodically determine the fluid application parameters and use these to automatically control the administration of fluid and/or medication, for example every four hours or at a similar rate. Thus, a closed-loop system may be provided. It is noted that, in order to determine the amount of fluid to be administered, the controller typically uses an estimate of the fluid output of the patient in addition to the target fluid balance. Even in a closed-loop system, this estimate is in practice often provided by the clinician, but may be measured automatically as well.

In other cases, the fluid is not administered automatically based on the determined target fluid balance in a fully closed-loop fashion, but in a process that involves the clinician. E.g., the system outputs a recommended target fluid balance and a clinician may be charged with determine the amount of fluid to be administered to achieve this balance. In this case, the provided techniques still provide automated control of the fluid balance, even if reaching this fluid balance is done by a human. It is also possible for the clinician to have a larger role, e.g., to also confirm or adapt the target fluid balance recommendation by the model.

Optionally, the determined target fluid balance may be displayed to a user, e.g., a clinician, for example on a patient monitor. This way, the clinician may ensure that an amount of fluid is administered according to the determined target fluid balance, or may possibly confirm or adapt the recommendation. To facilitate such decision making by the clinician, target fluid balances determined according to one or more trained fluid balance control models may be displayed by superimposing these target fluid balance values on a time series of measured fluid balances of the patient. A particularly effective visualization is to display a target dosage level range indicated by a target fluid balance as a "target box": a box superimposed on the time series. For example, fluid balance control models optimized for different objectives may be used. This addresses a central challenge in the application of artificial intelligence, namely, reasoning about the recommended action. Superimposing target values on the measured fluid balance enables easier interpretation of model's recommended actions and facilitates comparisons between different models. Previously recommended actions may be displayed as well, e.g., alongside the actual actions taken for the patient, thus enabling the user to make a more meaningful comparison. It is noted that the provided techniques do not diagnose any particular clinical picture but are instead aimed at the technical problem of assisting the controlling of fluid balance in an optimal way.

Optionally, the provided techniques may support multiple respective fluid balance control models trained for respective fluid application objectives. A selection of a fluid application objective may be obtained, and the fluid balance control model that is applied may be selected from the multiple models according to the obtained selection. The selected objective may change over time during a fluid application. This way, a fluid application that is appropriate for the situation at hand may be used, this obtaining a more optimal recommendation. The objective is typically selected by the clinician, but can also be derived automatically from patient data, e.g., based on an admission diagnosis of the patient or based on other information, e.g., retrieved from an electronic medical record (EMR).

Optionally, a selection by a user may be obtained of one or more patient criteria, for example interactively, and may be used to provide advise tailored to that selection of patient criteria. A dataset of past fluid application instances may be used, which may be filtered according to the selected criteria. Given one or more fluid balance control models, these models may be applied to the subset of patients to determine a target fluid balance for that subset (e.g., as a most commonly selected action, or as respective percentages of cases in which respective actions were selected). This allows the clinician to understand and reason about how the recommendations provided by the model are affected by different criteria. The selection of patient criteria can be implemented as an interactive decision table. This aspect addresses an important problem when using AI: how do we know that taking the recommended action will lead to an improvement? It also helps clinicians to reason about different recommendations provided by the respective AI models.

Optionally, the selection of the one or more patient criteria may be prepopulated according to the subsets of patients to which a current patient belongs, to make it easier to explore how the different subgroups to which the patient belongs, affect the recommendations by the model(s).

Optionally, the subset of patients may be further filtered according to the determined target fluid balance to obtain a further subset of patients. An outcome of the fluid therapy may be output for the further subset of patients. This way, it is enabled to explore potential outcomes if the physician follows the recommendations proposed by the model(s). The user can assess the model(s) on sub-populations of interest and quantify relevant patient outcomes based on different model decisions. This allows to assess what-if scenarios where different actions chosen on the same cohort lead to different patient outcomes.

The system described above may be implemented in various ways, examples of which are provided herein. The system may for example comprise a patient monitor, e.g., a bedside monitor, on which the determined target fluid balance is displayed. The displaying can also be performed on a central station, e.g., of a hospital information system. The system may involve an infusion pump which is used to automatically administer fluid and/or medication, e.g., in a closed-loop fashion or involving a clinician. The application of the fluid balance control model can take place on one of the abovementioned devices, e.g., the patient monitor or infusion pump, on a dedicated device, in a cloud environment, etc.

Further envisaged aspects are a training system and a computer-implemented training method for training a fluid balance control model for use as described above, based on a training dataset of past fluid application instances.

It will be appreciated by those skilled in the art that two or more of the above-mentioned embodiments, implementations, and/or optional aspects of the invention may be combined in any way deemed useful.

Modifications and variations of any computer-implemented method and/or any computer readable medium, which correspond to the described modifications and variations of a corresponding system, can be carried out by a person skilled in the art on the basis of the present description.

BRIEF DESCRIPTION OF THE DRAWINGS

These and other aspects of the invention will be apparent from and elucidated further with reference to the embodiments described by way of example in the following description and with reference to the accompanying drawings, in which:

Fig. 1 shows a system for training a fluid balance control model;
Fig. 2 shows a system for use in intravenous fluid application to a patient;
Fig. 3a shows states, actions, and rewards of a fluid balance control model;
Fig. 3b shows an example of how to apply a fluid balance control model;
Fig. 4 shows a detailed example of how to display a target fluid balance;
Fig. 5 shows a detailed example of how to display a target fluid balance;
Fig. 6 shows a computer-implemented method for use in intravenous fluid application to a patient;
Fig. 7 shows a computer-readable medium comprising data.

It should be noted that the figures are purely diagrammatic and not drawn to scale. In the figures, elements which correspond to elements already described may have the same reference numerals.

DETAILED DESCRIPTION OF EMBODIMENTS

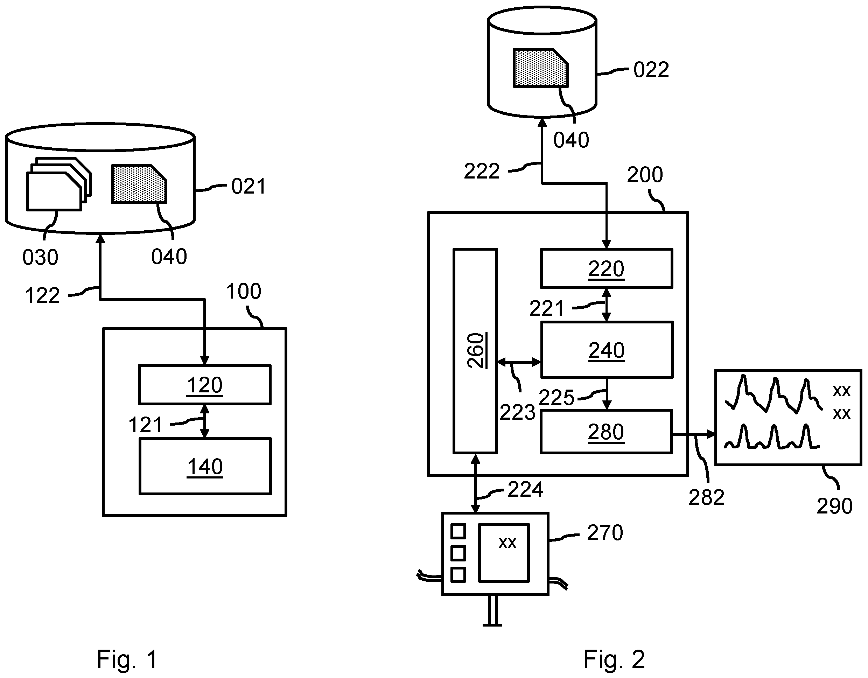

Fig. 1 shows a system 100 for training a fluid balance control model.

The system 100 may comprise a data interface 120 for accessing a training dataset of past fluid application instances. A past fluid application instance may comprise a past state of a patient, a past target fluid balance, and a reward value indicating a clinical outcome resulting from applying the past target fluid balance to the patient in the past state. Multiple past instances may correspond to different time points of the same fluid application session. For example, the total number of past fluid application instances used for training may be at most or at least 1000, at most or at least 10000, or at most or at least 100000.

Data interface 120 may be further for accessing model data 040 representing the fluid balance control model being trained. The fluid balance control model may be configured to determine a target fluid balance based on a state of the patient. The state may comprise measurements of one or more physiological parameters of the patient. The model data may comprise trainable parameters of the model, e.g., neural network weights and/or biases and the like. For example, the number of trainable parameters may be at most or at least 1000, at most or at least 10000, or at most or at least 100000. The model data 040 may be used to provide recommendations for intravenous fluid application according to a method described herein, e.g., by system 200 of Fig. 2.

For example, as also illustrated in Fig. 1, the input interface may be constituted by a data storage interface 120 which may access the data 030, 040 from a data storage 021. For example, the data storage interface 120 may be a memory interface or a persistent storage interface, e.g., a hard disk or an SSD interface, but also a personal, local or wide area network interface such as a Bluetooth, Zigbee or Wi-Fi interface or an ethernet or fiberoptic interface. The data storage 021 may be an internal data storage of the system 100, such as a hard drive or SSD, but also an external data storage, e.g., a network-accessible data storage. In some embodiments, the data 030, 040 may each be accessed from a different data storage, e.g., via a different subsystem of the data storage interface 120. Each subsystem may be of a type as is described above for data storage interface 120.

The system 100 may further comprise a processor subsystem 140 which may be configured to, during operation of the system 100, train the model 040 based on the training data 030, using any training technique suitable for the type of model 040. Training may be performed using stochastic approaches such as stochastic gradient descent, e.g., using the Adam optimizer as disclosed in Kingma and Ba, "Adam: A Method for Stochastic Optimization" (available at https://arxiv.org/abs/1412.6980 and incorporated herein by reference). As is known, such optimization methods may be heuristic and/or arrive at a local optimum. Training may be performed on an instance-by-instance basis or in batches, e.g., of at most or at least 64 or at most or at least 256 instances.

The system 100 may further comprise an output interface for outputting trained data 040 representing the learned (or 'trained') model. For example, as also illustrated in Fig. 1, the output interface may be constituted by the data interface 120, with said interface being in these embodiments an input/output ('IO') interface, via which the trained model data 040 may be stored in the data storage 021. For example, the model data defining the 'untrained' model may during or after the training be replaced, at least in part, by the model data of the trained model, in that the parameters of the model, such as weights and other types of parameters of neural networks, may be adapted to reflect the training on the training data 030. This is also illustrated in Fig. 1 by the reference numerals 040 referring both to trained and untrained model data in the data storage 021. In other embodiments, the trained model data may be stored separately from the model data defining the 'untrained' model. In some embodiments, the output interface may be separate from the data storage interface 120, but may in general be of a type as described above for the data storage interface 120.

Fig. 2 shows a system 200 that can be used for intravenous fluid application to a patient, in particular to provide recommendations for the fluid application and/or to perform the fluid application itself.

The system 200 may comprise a data interface 220 for accessing model data 040 representing a trained fluid balance control model. The fluid balance control model may be configured to determine a target fluid balance based on a state of the patient. The state may comprise measurements of one or more physiological parameters of the patient. The model data 040 may have been determined by system 100 of Fig. 1 or as described elsewhere. The system 200 may train the model in addition to applying it, e.g., may be combined with the system of Fig. 1.

For example, as also illustrated in Fig. 2, the data interface may be constituted by a data storage interface 220 which may access the data 040 from a data storage 022. In general, the data interface 220 and the data storage 022 may be of a same type as described with reference to Fig. 1 for the data interface 120 and data storage 021.

The system 200 may further comprise a processor subsystem 240 which may be configured to, during operation of the system 200, perform a management of the fluid application. That is, the system may determine a target fluid balance of a patient based on current measurements of that patient, e.g., periodically or upon request of a clinician. The processor subsystem may be configured to obtain the state of the patient. The obtaining of the state may comprise obtaining current measurements of one or more physiological parameters via a sensor interface 260, as also discussed elsewhere. Instead or in addition at least part of the state may be retrieved via data interface 220, e.g., from an electronic medical record of the patient (not shown), or the like. Processor subsystem 240 may be further configured to apply the fluid balance control model 040 to the state of the patient to determine the target fluid balance, and to output the determined target fluid balance. For example, processor subsystem 240 may be configured to perform the management of the fluid therapy periodically, for example at a rate of at most or at least once per hour, at most or at least once per four hours, and/or at a rate of at most or at least once per 24 hours.

It will be appreciated that the same considerations and implementation options apply for the processor subsystem 240 as for the processor subsystem 140 of Fig. 1. It will be further appreciated that the same considerations and implementation options may in general apply to the system 200 as for the system 100 of Fig. 1, unless otherwise noted.

In some embodiments, the system 200 may comprise a sensor interface 260 for obtaining sensor data 224 representing current measurements of one or more physiological parameters of the patient. For example, the sensor interface 260 is shown in the figure to be connectable to an infusion pump 270 that may provide measures of one or more physiological parameters, e.g., heart rate and/or blood pressure, of the patient. Instead of or in addition to the infusion pump, the sensor interface 260 may be connectable to another sensing device, such as a patient monitor and/or a blood pressure cuff for providing the measurements. Other types of physiological parameters and corresponding sensors are within reach of the skilled person. The sensor data interface 260 may have any suitable form corresponding in type to the type of sensor, including but not limited to a low-level communication interface, e.g., based on I2C or SPI data communication, or a data storage interface of a type as described above for the data interface 220. The sensing device can optionally be part of system 200.

In some embodiments, the system 200 may comprise an output interface for providing fluid application parameters to an infusion pump 270. In this particular example, the output interface is combined with sensor interface 260, but this is not needed. The fluid application parameters may indicate an amount of fluid and/or medication to be administered to the patient, for example determined by a controller implemented by processor subsystem 240 and configured to automatically control the administration of fluid and/or medication. The infusion pump can be part of system 200 or can be separate. For example, systems 200 and 270 of the figure can be combined in a single infusion pump device.

In some embodiments, the system 200 may comprise an output interface 280 to a rendering device, such as a display, a light source, a loudspeaker, a vibration motor, etc., which may be used to generate a sensory perceptible output signal which may be generated based the determined target fluid balance. For example, as shown in the figure, the system may comprise a patient monitor 290 on which the determined target fluid balance is displayed. For example, the system 200 and the patient monitor 290 can be incorporated into a single device. However, system 200 can also be separate from the patient monitoring device, with the output interface being used to provide the target fluid balance computed by the system 200 to the patient monitoring device.

In general, each system described in this specification, including but not limited to the system 100 of Fig. 1 and the system 200 of Fig. 2, may be embodied as, or in, a single device or apparatus, such as a workstation or a server. The device may be an embedded device. The device or apparatus may comprise one or more microprocessors which execute appropriate software. For example, the processor subsystem of the respective system may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the processor subsystem of the respective system may be implemented in the device or apparatus in the form of programmable logic, e.g., as a Field-Programmable Gate Array (FPGA). In general, each functional unit of the respective system may be implemented in the form of a circuit. The respective system may also be implemented in a distributed manner, e.g., involving different devices or apparatuses, such as distributed local or cloud-based servers. In particular, the system may comprise a server 200 that determines the target fluid balance, combined with an infusion pump 270, patient monitor 290, EMR, central station such as the eICU eCareManager portal and/or other user interface for applying the intravenous fluid application and/or showing the target fluid balance to a user.

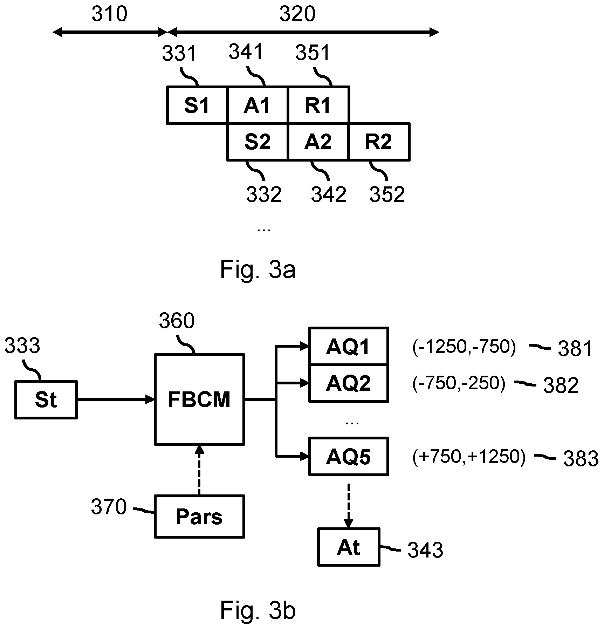

Fig. 3a shows a detailed, yet non-limiting, example of states, actions, and rewards of a fluid balance control model.

The figure schematically illustrates two phases of a fluid management. Shown is a resuscitation phase 310 in which e.g. fluid is administered containing vasopressors to raise blood pressure, or the like. Also shown is a de-resuscitation phase 320 in which fluid is to be removed. In this example, the fluid balance control model is configured to provide fluid therapy recommendations in the de-resuscitation phase 320.

As illustrated, the patient stay may be divided into state/action/reward blocs of fixed durations, e.g., of 4 hours or similar, e.g., at most or at least 1 hour, at most or at least 4 hours, or at most or at least 24 hours. The control model may use current measurements corresponding to the state block, e.g. of 4 hours, to recommend a target fluid balance during the action block, e.g., the next 4-hours, such that a reward is optimized for the following reward block, e.g., the following 4-hours. For example, the figure shows consecutive state block **S1**, 331, action block **A1**, 341, and reward block **R1**, 351. The figure further shows consecutive state block **S2**, 332, action block **A2**, 332, and reward block **R2**, 352. As shown in the figure, the state block **S2** temporally following a previous state block **S1**, may temporally correspond to the action block **A1** for the previous state block **S1**, and similarly, the action block **A2** for the consecutive state block **S2** may temporally correspond to the reward block **R1** for the previous state block, etcetera.

For example, states **Si**, actions **Ai**, and rewards **Ri** may be comprised in a training dataset of past fluid application instances as used to train the fluid balance control model. Here, a past fluid application instance **Si**, **Ai**, **Ri**, e.g., **S1**, **A1**, **R1** or **S2**, **A2**, **R2**, may comprise a past state **Si** of a patient, a past target fluid balance **Ai**, and a reward value **Ri** indicating a clinical outcome resulting from applying the past target fluid balance **Ai** to the patient in the past state **Si**. In particular, the dataset of past fluid application instances may be denoted as a set of offline state-transition tuples

$$\{(s_1, a_1, s_1', r_1), \cdots, (s_N, a_N, s_N', r_N)\}$$

, where $s_n$ is the current patient state $s_n$, **Si**, $a_n$ is applied action **Ai**, $s_n'$ is the new patient state **Si+1** after action $a_n$ was applied and $r_n$ **Ri** is the corresponding reward associated with the state transition $p(s_n' | s_n, a_n)$.

Generally, a state **Si** that is input to the fluid balance control model, may comprise measurements of one or more physiological parameters of a patient. For example, the physiological parameters can include one or more of the following, e.g., at least five, at least ten, or at least 20 parameters. For example, the physiological parameters may comprise at least 5, at least 10, or at least 20 parameters be selected from:

ADMISSION_WEIGHT, DIASTOLIC_BP, IS_VENTILATED, RESP_RATE, AGE, DIURETICS_INPUT, LACTATE, SAO2, AKI, GCS_TOTAL, MAGNESIUM, SODIUM, ALBUMIN, EOS, MAWP, SOFA, ANION_GAP, FIO2, MEAN_BP, SPO2, APACHE, FLUID_BALANCE_ABSOLUTE, OUTPUT, SURGERY_BEFORE_ICU, AST, FRESH_FROZEN_PLASMA, PACKED_RED_BLOOD_CELLS, SUSPECTED_INFECTION, BANDS, GLUCOSE, PACO2, SYSTOLIC_BP, BASE_EXCESS, HAD_OXYGENTHERAPY, PAO2, TEMP_C, BASOPHILS, HEMATOCRIT, PCO2, TIDAL_VOLUME, BICARBONATE, HEMOGLOBIN, PEEP, TIME _SINCE_VASOPRESSOR_OFFSET, BILIRUBIN, HR, PH, WAS_EXTUBATED, BUN, ICUADMISSION_NO, PIP, WAS_IN_SURGERY, CALCIUM, INPUT, PLATELET, WAS_SELFEXTUBATED, CHLORIDE, IONIZED_CALCIUM, POTASSIUM, WBC, CO2, IS_CRRT DIALYSED, PTT, WEIGHT KG, CREATININE, IS READMISSION, RATIO_PAO2_FIO2.

Values for at least some of these physiological parameters may be obtained via a sensor interface. It is also possible for some of these values to be manually entered by a user, or to be accessed via an EMR, however. Performing the measurements accessed via the sensor interface may involve measurement tasks being performed by humans. Multiple measurements of a physiological parameter made during the time window of the state Si may be available, in which case they may be aggregated, e.g., by taking the median. More typically, however, only one measurement will have been made during the time window, or none at all, in which case the most recent measurement preceding the end of the time window may be used. For example, one of the physiological parameters may be a lactate value, which is typically measured only once every 24 hours.

In particular, the state Si may comprise patient demographics, vital sign measurements, laboratory measurements, ventilator settings, past intervention information, comorbidity indicators, and/or fluid balance history information. For example, the patient demographics may include age and/or weight. The vital sign measurements may comprise heart rate and/or blood pressure. The laboratory measurements can indicate measurements from one or more of basic metabolic panel, liver panel, white blood cells panel, red blood cells panel, coagulation, and blood gas variables. The ventilator settings can include FiO2 and/or mean airway pressure. The past intervention information can include information about surgery and/or blood transfusion.

For example, the feature vector may contain one or more of the following features, for which exemplary data types and explanations are given:

| Feature | Pref? | Type | Explanation |
|---|---|---|---|
| ADMISSION_WEIGHT | | 1 | float64 Admission weight in kg (static column) |
| AGE | 1 | float64 | Age of the patient in years |
| AKI | 1 | float64 | AKI index of the patient |
| ALBUMIN | 1 | float64 | |
| ANION_GAP | 1 | float64 | |
| APACHE | 1 | float64 | |
| AST | 1 | float64 | |
| BANDS | 1 | float64 | |
| BASE_EXCESS | | 1 | float64 |
| BASOPHILS | 1 | float64 | |
| BICARBONATE | | 1 | float64 |
| BILIRUBIN | 1 | float64 | |
| BINNED_FLIOD_ | BAL_ | ABS | float64 Absolute fluid balance (=total milliliters) binned to |

500ml intervals (centered around 0), e.g., range (-250ml,250ml] is mapped to 0; (250ml, 750ml] to 500ml etc.
BINNED_FLUID_BALANCE_ABS_DELTA_SINCE_LAST_BLOC

| | | float64 | Abs. fluid balance delta from the beginning to the end of this bloc |

(last fl. bal. value minus first fl. bal. value in ml); binned to 500ml intervals (centered around 0)

| | | | |
|---|---|---|---|
| BUN | 1 | float64 | |
| CALCIUM | 1 | float64 | |

(continued)

| Feature | Pref? | Type | Explanation |
|---|---|---|---|
| CHARTTIME | | int64 | End time of bloc in minutes; as UNIX timestamp |
| CHARTTIME_BLOC_START | | | float64 Start time of bloc in minutes; as UNIX timestamp |
| CHLORIDE | 1 | float64 | |
| CO2 | 1 | float64 | |
| CREATININE | 1 | float64 | |
| CRRT_DURATION | | | float64 Total CRRT duration in an ICU stay in minutes |
| CRRT_FREE_DAYS | | | float64 Total amount of CRRT-free days (decimal). CRRT-free periods need to last at least 2 days (>=48h) to be counted into the CRRT free days |
| DIASTOLIC | BP | 1 | float64 |
| DIURETICS | _INPUT | 1 | float64 Diuretics given to a patient in milligrams during bloc |
| DIURETICS_ PRESCRIBED | | float64 | Prescribed diuretics in mg during an ICU stay |
| DIURETICS_PR_ DURATION | | float64 | Duration of diuretic prescriptions in minutes. |
| DRIVING_ PRESSURE | | float64 | |
| EOS 1 | | float64 | Eosinophil Count |
| ETHNICITY | | int64 | Ethnicity of patient: unknown/other/native/white/... |
| EXP_30DAYS_ AFTER_HADM | | int64 | 1 if patient died in 30 days after hospital admission |
| EXP_90DAYS_ AFTER_HADM | | int64 | 1 if patient died in 90 days after hospital admission |
| EXPIRED_IN_ HOSPITAL | | int64 | 1 if patient expired in ICU |
| EXPIRED_IN_ ICU | | int64 | 1 if patient expired in hospital |
| FIO2 1 | | float64 | |
| FLUID_ BALANCE_ABS | | 1 | float64 Absolute fluid balance at the end of this bloc in ml. |
| FLUID_BALANCE_ABSOLUTE_DELTA_SINCE_LAST_BLOC | | | |
| | | float64 Absolute to the end | fluid balance change from the beginning of the bloc. In milliliters. |
| FLUID_BAL_ NORMALIZED | | float64 | Absolute fluid balance at the end of this bloc in ml |
| FRESH_ FROZEN_PL | 1 | float64 in this bloc. | Amount of fresh frozen plasma given to the patient In milliliters. |
| GCS_EYES 1 | | float64 | |
| GCS MOTOR 1 | | float64 | |

(continued)

| Feature | Pref? | Type | Explanation | |
|---------|-------|------|-------------|---|
| GCS_VERBAL1 | float64 | | | |
| GENDER | int64 | Gender of | the person. 0 for male; 1 for female | |
| GLUCOSE 1 | float64 | | | |
| HADM_ID | int64 | Hospital | admission ID | |
| HAD_FLUID_ INP_ | CARTINGS_ float64 | BEFORE_ 1 if patient | ADMISSION has charted fluids before ICU admission | |
| HAD_FLUID_ OUT_ | CARTINGS_ float64 | BEFORE_ 1 if patient | ADMISSION has charted outputs before ICU admiss. | |
| HAD_ OXYGENTHPY | 1 | float64 | 1 if patient has oxygentherapy in this bloc | |
| HAD_PREADM_ INP_CHART | | float64 | 1 if explicit pre-admission/surgical fluid chartings | |
| HAD_PREADM_ OUT_CHART | | float64 | 1 if explicit pre-admission/surgical output chartings | |
| HEMATOCRIT | 1 | float64 | | |
| HEMOGLOBIN | 1 | float64 | | |
| HOSPITAL_ ADMISSION_ TIME | | float64 | UNIX timestamp of the hospital admission in mins | |
| HOSPITAL_ DISCHARGE_TM | | float64 | UNIX timestamp of the hospital discharge in mins | |
| HR 1 | float64 | | | |
| ICUADMISSION_ NO | 1 | float64 | Admission number of this ICU stay (starts at 0) | |
| ICUSTAY_ID | | int64 | The ICU | stay's identifier |
| ICU_ ADMISSION_ TIME | | | | float64 The ICU admission time |
| ICU_ ADMISSION_ UNIT in mins | | | | object The UNIX timestamp of the ICU admission |
| ICU_ DISCHARGE_ TIME | | | float64 | The UNIX timestamp of the ICU discharge in mins |
| INPUT | 1 | float64 | | Total input in milliliters administered during bloc |
| IONIZED_ CALCIUM | 1 | | float64 | |
| IS_CRRT_ DIALYSED | 1 | | float64 | True/1 if patient has CRRT during this bloc |

(continued)

| Feature | Pref? | Type | | Explanation |
|---|---|---|---|---|
| IS_READMISSION | | 1 | int64 | True/1 if this ICU stay is a readmission |
| IS_VENTILATED | | 1 | float64 | True/1 if mechanical/ invasive ventilation in this bloc |
| LACTATE | 1 | float64 | | |
| LOS_HOSPITAL | | | float64 | Length of the hospital stay in minutes |
| LOS_ICU | | | float64 | Length of the ICU stay in minutes |
| MAGNESIUM | 1 | float64 | | |
| MAWP | 1 | float64 | | |
| MEAN_BP | 1 | float64 | | |
| OUTPUT | 1 | float64 | | Total output during this bloc. In milliliters. |
| PACKED_RED_CELLS | | 1 | float64 | Packed red blood cells given to patient in bloc, ml |
| PACO2 | 1 | float64 | | |
| PAO2 | 1 | float64 | | |
| PCO2 | 1 | float64 | | |
| PEEP | 1 | float64 | | |
| PH | 1 | float64 | | |
| PIP | 1 | float64 | | |
| PLATEAU VOLUME | | 1 | float64 | |
| PLATELET | 1 | float64 | | |
| POTASSIUM | 1 | float64 | | |
| PTT | 1 | float64 | | |
| RATIO_PAO2_FIO2 | | 1 | float64 | Ratio between PAO2 and FIO2, both features taken |
| READMISSIONS | | | float64 | Total number of readmissions of a patient |
| RESP RATE | 1 | float64 | | |
| SAO2 | 1 | float64 | | |
| SODIUM | 1 | float64 | | |
| SOFA | 1 | float64 | | |
| SPO2 | 1 | float64 | | |
| SUBJECT_ID | | int64 | | Patient's identifier |
| SURGERY_BE_ICU | | 1 | float64 | 1 if patient had a surgery before ICU administration |
| SURGERY_DU_ICU | | float64 1 if patient had a surgery during ICU administration | | |

(continued)

| Feature | Pref? | Type | Explanation |
|---|---|---|---|
| SUSPECT INFECTION | 1 | float64 1 if patient has a suspected infection during bloc = antibiotics 72h after/24h before microbiology event | |
| SYSTOLIC BP | 1 | float64 | |
| TARGET NEXT BLOC BINNED | | FLUID BALANCE ABS DELTA | |
| | float64 | Next bloc's binned absolute fluid balance difference. In 500 ml | |
| bins (0, +/- 500ml, +/- 1000ml etc.). | | Each bin is centered around the actual label (e.g. 250 to 750ml | |
| corresponds to 500ml) | | | |
| TARGET NEXT BLOC FLUID | | BALANCE ABS DELTA | |
| | float64 | Next bloc's absolute fluid balance difference, real | |
| TEMP_C 1 | float64 | | |
| TIDAL VOLUME | 1 | float64 | |
| TIME_SINCE_ VASOP | 1 | int64 Time since vasopressor offset in minutes (start time design matrix) | of |
| the first bloc for each patient in the | | | |
| VASO_ADMIN_ END | | int64 Time where administration of vasos/ inotr. stopped (UNIX charttime in minutes) | |
| VASO_ADMIN_ START | | int64 Time where administration of vasos/ inotr. started (UNIX charttime in minutes) | |
| VENT_ DURATION | | float64 Ventilation duration during the total ICU stay, mins | |
| VENT_FREE_ DAYS | | float64 Number of ventilation free days. Starts counting if the hours (2 days) long. | |
| vent-free period has been at least 48 | | | |
| WAS_ EXTUBATED | 1 | float64 1 if ongoing extubation in this bloc. | |
| WAS_HAVING_ SHOCK | | float64 1 if patient had septic shock during the | ICU |
| stay | | | |

(continued)

| Feature | Pref? | Type | Explanation |
|---|---|---|---|
| WAS_IN_SURGERY | 1 | float64 True/1 if patient is in surgery during this bloc. | |
| WAS_SELFEXTUB | 1 | float64 True/1 if patient is self-extubated in this bloc | |
| WBC 1 | float64 | | |
| WEIGHT_KG 1 | float64 | Most recent patient weight in kilograms | |

Here, the features marked with "Pref?" were found to work particularly well. In particular, the inventors achieved good results with a model trained using all features marked with "Pref?". However, it is also possible to use a subset of these features and/or to replace these features by others. For example, at least 10, at least 20, or at least 50 of the above features may be used, e.g., at least 10, at least 20, or at least 50 of the above preferred features.

The action $\mathbf{Ai}$ may represent a target fluid balance over a next time window, in other words a discretized dosage level of the fluid balance within the time window. The target fluid balance may be indicated relative to a current fluid balance, for example, by indicating a target dosage level range ΔFluidBalance for a following time period, e.g., zero, negative high/low, positive high/low. Accordingly, the action $\mathbf{Ai}$ may represent by how much the fluid balance is to be increased or decreased over the time window, e.g., the next four hours. As a concrete example, the action may be selected as one of the following five ranges: {(-1250, -750), (-750, -250), (-250, 250), (250, 750), (750, +1250)}. It is possible for the action to also indicate additional aspects of the fluid application, such as whether the patient should stay on ventilation or put off. For example, combined with the five ranges above this can give ten possible actions: (-1250, -750) with ventilation; (-1250, -750) without ventilation; (-750, -250) with ventilation, etc.

The reward $\mathbf{Ri}$ may indicate a clinical outcome resulting from applying the past target fluid balance $\mathbf{Ai}$ to the patient in the past state $\mathbf{Si.}$ Here, the clinical outcome can indicate an organ system function, various examples of which are given below. The reward may combine several organ system function indicators and/or may include more general health indicators such as a readmission rate and/or a mortality rate, e.g., in a weighted sum.

As a concrete example, consider an example where a physician monitors lactate to track effectiveness of a therapy. In this example, the reward may be defined as a combination of a readmission reward, a mortality reward, and a lactate reward, e.g., defined as a weighted sum as in the following example:

$$0.3*\text{Readmission} + 0.3*\text{Mortality} + 0.4*(\Delta\text{Lactate} \geq 10\% \text{ decrease})$$

Several concrete clinical outcome indicators are now discussed that can be used e.g. to indicate organ system function for use in the reward function.

**1) Lactate level**

Lactate may be used to capture whether the administration of fluids can achieve lactate clearance. The reward may be configured to encourage lactate clearance and keeping low lactate level. For example, high reward may be assigned to lactate clearance and to keeping low lactate level, with other transactions not awarded, for example:

| Old lactate value | Change in lactate value | Reward |
|---|---|---|
| >4 | Decrease: >10% | 1 |
| >4 | Decrease: <10% | 0.8 |
| >4 | 0 | 0 |
| >4 | Increase: >10% | 0 |
| >4 | Increase: <=10% | 0 |
| <=4 | Decrease: <10% | 1 |
| <=4 | Decrease: >10% | 1 |
| <=4 | 0 | 1 |
| <=4 | Increase: <10% | 0 |
| <=4 | Increase: >10% | 0 |

**2) Hemodynamic stability**

A reward value indicating hemodynamic stability may be derived from the shock index (heart rate divided by systolic blood pressure). The reward may be configured to encourage decreasing shock index and keeping low shock index. As an illustrative example, the shock index may be discretized into four discrete states listed below. This discretization method is derived from clinical literature on the association between the shock index level and mortality: higher shock index value is associated with higher mortality.

| Shock index range | <0.6 | [0.6,1] | [1,1.4] | >1.4 |
|---|---|---|---|---|
| Discretized state | $S_1$ | $S_2$ | $S_3$ | $S_4$ |

The reward may be defined such that high reward is assigned to decreasing shock index or keeping low shock index level, while other transitions are not encouraged, e.g.:

| Old shock index | New shock index | Reward |
|---|---|---|
| $S_1$ | $S_1$ | 1 |
| $S_2$ | $S_2$ | 0.6 |
| $S_3$ | $S_3$ | 0.2 |
| $S_4$ | $S_4$ | 0 |
| Any state | Higher state | 0 |
| Any state | Lower state | 1 |

**3) Renal function**

The acute kidney injury (AKI) score may be used to characterize the renal function. The reward may encourage decreasing AKI value or keeping normal AKI value (AKI=0), e.g.:

| Old AKI value | New AKI | value | Reward |
|---|---|---|---|
| 0 | 0 | 1 | |
| >0 | Lower state | | 1 |
| Any stage | Higher state | | 0 |
| 1 | 1 | 0.4 | |
| 2 | 2 | 0.2 | |
| 3 | 3 | 0 | |

**4) Respiratory function**

The PF ratio may be used to characterize the respiratory function, e.g., fluid overload could cause edema. The reward function can for example be derived from the PF ratio discretized following the Berlin definition as known per se:

| PF ratio range | >300 | (200,300) | (100,200) | <=100 |
|---|---|---|---|---|
| Discretized state | Normal $S_1$ | Mild $S_2$ | Moderate $S_3$ | Severe $S_4$ |

The reward may encourage transitioning to a lower state and keeping in the normal state. Other transitions can be penalized, e.g.:

| Old PF ratio | New PF | ratio | Reward |
|---|---|---|---|
| $S_1$ | $S_1$ | 1 | |
| $S_2$ | $S_2$ | 0.6 | |
| $S_3$ | $S_3$ | 0.2 | |
| $S_4$ | $S_4$ | 0 | |
| Any state | Higher state | | 0 |
| Any state | Lower state | | 1 |

**5) Mortality rate**

A reward based on a subgroup mortality rate may be used to characterize the long-term effect of fluid management on patient survival. The subgroup mortality rate may be determined by assigning the patient to a subgroup based on the state **Si+2** of the patient resulting from applying the past target fluid balance **Ai** from state **Si,** and obtaining a mortality rate of the assigned subgroup. For example, clustering analysis may be applied on patient state vectors to identify patient subgroups with high risk of morality and low risk of mortality. A given patient state **Si+2,** may be assigned to one of the subgroups defined by the clustering. The corresponding mortality rate mortalityRate may be used define the reward function e.g. as $r_{mort}$ = 1 — mortalityRate. Generally, the patient state used to derive the subgroup mortality rate reward may have the same features as the state to which the fluid balance control model is applied, except that the state **Si+2** resulting from applying the action **Ai** is used as opposed to the state **Si** from which a recommended action **Ai** is derived.

An alternative approach to determine a reward value for a given clinical outcome is as follows. This approach can be used for any value *v* representing a clinical outcome where higher values indicate worse patient outcomes (and, by negating the value, also where higher values indicate better patient outcomes). For example, it can be used for the lactate, shock index, AKI, ventilation status, and mortality rate rewards discussed above. The reward function maybe defined as follows:

$$R = 1 - \frac{v - v_{min}}{v_{max} - v_{min}}$$

where R is the reward function value, *v* is the outcome value after the action was taken, and $v_{max}$ and $v_{min}$ are maximal and minimal outcome values, e.g., measured across a patient population. For the PF ratio, the value can be negated, or equivalently, the reward can be defined as $\frac{v - v_{min}}{v_{max} - v_{min}}$ .

Fig. 3b shows a detailed, yet non-limiting, example of how to apply a fluid balance control model.

The figure shows a fluid balance control model **FBCM,** 360. The model **FBCM** may be configured to determine a target fluid balance **At,** 343, based on a state **St,** 333, of the patient. For example, the state **St** and the target fluid balance **At** may be as discussed with respect to Fig. 3a.

In general, the target fluid balance may be determined from respective suitability values indicating the suitability of respective possible target fluid balances for the state **St.** Shown by way of example are suitability values **AQ1,** 381, **AQ2,** 382, up to **AQ5,** 383, for respective target fluid balance (-1250,-750); (-750,-250); up to (+750,+1250). Depending on the type of machine learning model used, the suitability values can for example be classification scores, Q-function values, etc. Examples are given herein.

As shown, the fluid balance control model **FBCM** is typically parameterized by a set of trainable parameters **PARS,** 370, for example, comprising neural network weights and/or biases or similar. For example, the model **FBCM** may comprise a neural network. Neural networks are also known as artificial neural networks. Examples include deep neural networks and convolutional neural networks. In this case, the set of parameters **PARS** may comprise weights of nodes of the neural network. For example, the number of layers of the model may be at least 5 or at least 10, and the number of nodes and/or weights may be at least 1000 or at least 10000. Various known architectures for neural networks and other types of machine learnable models may be used. It is beneficial from the point of view of efficiency of training to use a model **FBCM** which is amenable to gradient-based optimization, e.g., which is continuous and/or differentiable in its set of parameters **PARS.**

Generally, the fluid balance control model may be trained on a training dataset of past fluid application instances. A past fluid application instance may comprise a past state of a patient, a past target fluid balance, and a reward value indicating a clinical outcome resulting from applying the past target fluid balance to the patient in the past state. Such past training instances may be seen as a set of offline state-transition tuples $\{(s_1, a_1, s'_1, r_1), \cdots, (s_N, a_N, s'_N, r_N)\}$ ,

where $s_n$ is the current patient state $s_n$, **Si**, $a_n$ is action **Ai** applied, $s'_n$ is the new patient state **Si+1** after action $a_n$ was applied and $r_n$ **Ri** is the corresponding reward associated with the state transition $p(s'_n | s_n, a_n)$ . The fluid balance control model **FBCM** may be referred to as a fluid management policy. Training may comprise learning an optimal fluid management policy $\pi(a|s)$, **FBCM** which maps the patient state s, **St** to the action a, **At.** Such an optimal fluid management policy **FBCM** may lead to improved patient outcomes, in particular improved short-term functional outcomes (such as lactate clearance and hemodynamic stability) and/or long-term outcomes (such as survival and shorter length of stay).

Various possibilities are possible to implement the fluid balance control model **FBCM.** In principle, any AI model providing suitable outputs **At** can be used. It is also possible to use several fluid balance control models and e.g. output their respective recommendations to the user. Several specific examples are now provided.

**Behavior cloning**

In this example, the fluid balance control model **FBCM** is trained on a labelled training dataset of patient states and associated physician-selected target fluid balances. In other words, the fluid balance control model **FBCM** is a physician policy indicating what action would be taken by an average physician. The model can be trained as a supervised classification model $\mu(a|s)$ from the training dataset $D_{BC}$ = {($s_1$, $a_1$), ..., ($s_N$, $a_N$)}. For example, the classification model can be a logistic regression model, a neural network, a gradient boosting model, etc. Optionally, class imbalance between different action classes may be dealt with by applying class-balanced sample reweighting during model training. The model may output respective predicted probabilities **AQi** for respective actions, e.g., target fluid balances. In use, the predicted class, e.g., the recommended target fluid balance **At,** may be selected as the class with maximal predicted probability **AQi.** As a concrete example, multiclass XGBoost with class weighting may be used to implement this model.

**Contextual bandits**

This example is similar to the previous example, but includes reweighting the past fluid application instances based on their reward values by applying inverse propensity scoring (IPS). Thus the model is encouraged to output actions with higher rewards. This model can be implemented using multiclass XGBoost with IPS, for example. Effectively, this model may be encouraged to output the optimal action that maximizes a reward. In inverse propensity scoring, past instances may be reweighed according to the proportion of the reward versus the predicted probability of choosing correct action by the physician policy discussed above. Thus, different instances may be treated with different importance weights, rather than the standard formulation of equal importance weight 1 on every instance.

The above is an example of using contextual bandit training to learn an optimal policy $\pi_{CB}(a|s)$ to maximize the cumulative reward overtime. In this example, offline contextual bandit training is implemented as a cost-sensitive classification. In particular, given the training dataset $D_{CB}$ = {($s_1$, $a_1$, $r_1$), $\cdots$, ($s_N$, $a_N$, $r_N$)}, the weight of the n-th sample may be computed as:

$$w_n = \frac{r_n}{\mu(a_n|s_n)}$$

where $\mu(a_n|s_n)$ represents the behavior policy learned through the behavior cloning model. During training, sample reweighting with weights $W_n$ may be applied. In use, the predicted probability **AQi** may be adjusted by dividing by the prior prevalence of the respective action classes. The prior prevalence may be computed from the total weights of each action class in the training data.

The above is a particular example of training the fluid balance control model by means of contextual bandit training on the training dataset of previously applied actions and computed awards. Other types of contextual bandit training can be used to optimize for the reward as well. Generally, contextual bandit training allows to learn to select optimal actions with respect to given rewards based on a training dataset where the optimal action was not necessarily taken. Contextual bandit training is also known as multi-world testing, associative bandits, learning with partial feedback, learning with bandit feedback, bandits with side information, multi-class classification with bandit feedback, associative reinforcement learning, or one-step reinforcement learning.

**Fitted Q-iteration**

In this example, the fluid balance control model **FBCM** is trained by Q-learning. Generally, Q-learning aims to learn a Q function $Q(s, a)$ to maximize the cumulative discounted reward over time. Applying the fluid balance control model **FBCM** may comprise evaluating the Q-function on the current state and the target fluid balance to obtain respective Q-function values **AQi,** and selecting the target fluid balance **At** based on the respective Q-function values **AQi.**

In particular, the model may be trained by fitted Q-iteration. Given the training dataset $\mathcal{D}_{FQI} = \{(s_1, a_1, s_1', r_1), \cdots, (s_N, a_N, s_N', r_N)\}$, the Q function may be initialized to zero. The training may involve an iterative procedure, wherein, in an iteration, a regression model $f_\theta(t)$ is trained to map the state-action tuple ($s$, $a$) to a target defined in a Bellman optimality condition. In particular, the following optimality condition may be used:

$$y = r + \gamma \cdot \max_a Q^{(t-1)}(s', a)$$

where $Q^{(t-1)}$ is the Q function learned in the previous iteration, and y is a discounting factor. The iterative procedure may be performed until the Q function values stabilize. In use, for a given state $s^*$, **St,** the predicted action **At** may be selected as the action associated with the largest Q-function value **AQi** when varying the action,

$$a^* = \operatorname*{argmax}_a Q(s^*, a).$$

In various embodiments, multiple respective fluid balance control models can be used. For example, respective fluid balance control models may be trained for respective fluid application objectives, e.g., rewards as discussed with respect to Fig. 3a, and/or may be trained using respective training techniques as discussed above. In particular, a selection of a fluid application objective may be obtained from a user or otherwise, based on which model(s) corresponding to that fluid application objective (e.g., optimize for lactate, shock index, AKI value, or PF ratio, optionally in combination with admission and/or mortality rewards), may be selected and applied. The fluid therapy objective can also be determined automatically, for example based on an admission diagnosis of the patient.

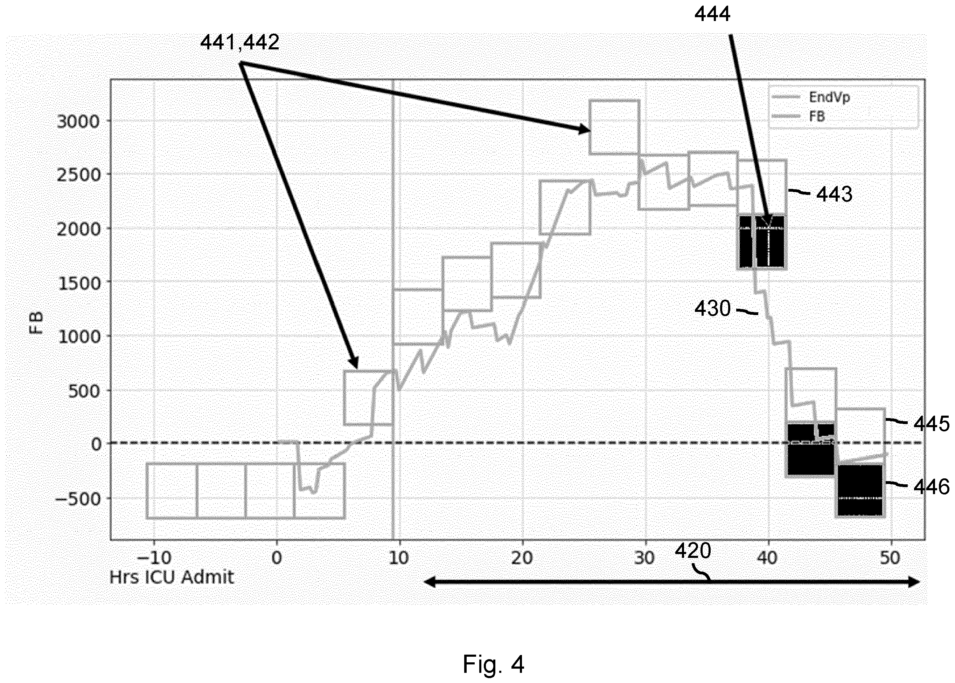

Fig. 4 shows a detailed, yet non-limiting, example of how to display a target fluid balance. The figure shows an example time series 430 of measured fluid balances of a patient, as may be shown on a patient monitor or on a web interface, for instance. The figure shows the fluid balance of a patient for 50 hours starting from ICU admission. As shown in the figure, in this example, the de-resuscitation phase 420 started around 10 hours from admission.

Target fluid balances determined according to one or more trained fluid balance control models may be presented to a user by superimposing these values on the time series 430.

The shown target fluid balances may be recommendations for the future, e.g., may correspond to a time span for which no time series measurements 430 are available. This way, the clinician can see the recommendations for the future and compare them to the time series available so far. This is the case for target fluid balances 445, 446 for example. Instead or in addition, presented target fluid balances may correspond to one or more time spans in the past, e.g., time spans for which also measurements 430 of the actually achieved fluid balance are shown. This way, target fluid balance(s) may be compared to each other and to the action that was actually taken. This is the case in the figure for target fluid balances 441-444.

In this example, a target fluid balance 441-446 is visualized as a so-called "target box". This means that a target dosage level range is indicated on the display as a box superimposed on the time series. One dimension of the box, e.g., the vertical dimension, may represent a recommended range for the fluid balance. Another dimension of the box, e.g., the horizontal dimension, may represent the time span to which the recommended range applies.

Differently visualized boxes e.g., with different border color, border line style, shading color, shading style, etc., may represent recommendations by different models (e.g., different types of models and/or models optimized with respect to different rewards). For example, target boxes 443, 445 may represent physician recommendations of the behavior cloning model of Fig. 3b, whereas target boxes 444, 446 may represent recommendations of a reward-based model, such as the contextual bandits or fitted Q-iteration models of Fig. 3b (indicated in this figure using a dark background). In case several models recommend the same action, a separate visualization may be used; the different recommendations may each be shown; or a single recommendation can be shown, for example. In this example, the physician recommendations 441, 442 agree with the reward-based recommendation, and in this case, only the physician recommendations are shown. In this example, the reward-based recommendations 444, 446 turn out to be more aggressive at recommending lower fluid balance during the de-resuscitation phase than the physician recommendations 443, 445.

Overall, the target box provides an intuitive visualization for a physician aiming to bring the fluid balance time series 430 to the target box by either giving more fluids or removing fluids. The target box method enables the user to visualize over the timeline of a selected patient, the actions recommended by the model(s). This is shown alongside the actual actions taken for the patient so the user can make a meaningful comparison. Thereby, the target box addresses a challenge that is central to the use of AI, namely, to allow reasoning about the recommended action.

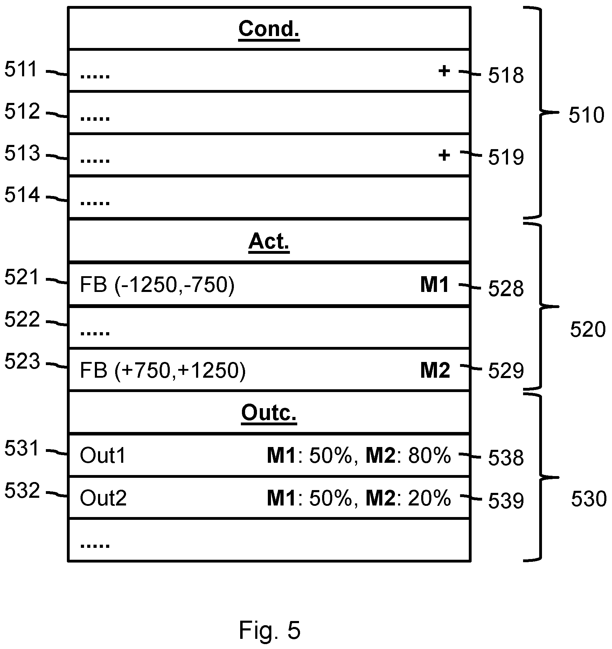

Fig. 5 shows a detailed, yet non-limiting, example of how to display a target fluid balance. This example schematically illustrates the use of an interactive decision table.

To use the interactive decision table, a user may provide a selection of one or more patient criteria. The respective patient criteria may define respective pre-defined cohorts of patients. The figure shows a list 510 of predefined criteria. For illustration, four criteria 511, 512, 513, 514 are shown that each may be individually selected or deselected. In the example shown, criteria 511 and 513 are selected as illustrated by markers 518, 519. The selection of the one or more patient criteria may be prepopulated according to a current patient, e.g., according to EMR data of a patient currently under fluid application.

Various criteria may be defined, e.g., by clinical experts, through literature review, and using machine learning, e.g., by applying feature selection. For example, one or more of the criteria may be selected from the group: obesity, high hemodynamic risk, low hemodynamic risk, high respiratory risk, respiratory acidosis, metabolic acidosis, ventilated, dialysis, decreasing fluid balance, increasing fluid balance, phenotype shock + hypoxemia, phenotype stable. A condition may be defined in terms of commonly observed features including, age, heart rate, systolic BP, diastolic BP, mean BP, shock index, respiratory rate, SpO2, temperature, haematocrit, haemoglobin, lactate, pH, PaCO2, PaO2, bicarbonate, base excess, albumin, blood urea nitrogen, creatinine, bilirubin, platelet, glucose, indicator variables for ventilation status. These features are generally sufficient to describe hemodynamic and respiratory status like shock or metabolic acidosis.

The selected criteria may be used to filter a dataset of past fluid application instances, thus obtaining a subset of patients satisfying the one or more patient criteria. For example, the patients in the dataset may have binary flags representing whether they belong to the each of the pre-defined cohort lists, which can be used to implement the filtering.

One or more respective fluid balance control model(s), e.g., different types of models and/or models optimized for different rewards, may be applied to the subset of patients to determine respective recommended target fluid balances for the subset of patients. For example the recommendation of a model for the subset of patient may be determined by majority vote, a top-2 or top-3 vote, as a probability distribution over possible actions, etc. To make the system more responsive to user selections and to increase overall efficiency, the model recommendations may be pre-calculated. The set of models being applied may be selected by the user.

The recommended actions of the respective models may be output. For example, the figure shows possible actions 520 for the target fluid balance, namely actions 521, 522, 523. In this example, action 521 is recommended by a model **M1,** as indicated by marker 528, whereas action 523 is recommended by another model **M2,** as indicated by marker 529. For example, the recommended actions can be "increase fluid balance", "decrease fluid balance", or "no change in fluid balance", possibly in combination with using or not using ventilation. Further examples are discussed with respect to Fig. 3a.

It is further possible to augment the interactive decision table functionality by exploring expected outcomes if a given target fluid balance is adopted. To this end, the subset of patients selected according to criteria 510, may by further filtered according to an action, e.g., action 521 recommended by model **M1** or action 523 recommended by model **M2.** This way, a further subset of patients may be obtained, for which an outcome of the fluid therapy may be determined based on the dataset.

For example, the figure shows outcomes 531, 532, e.g., "high hemodynamic risk", "high respiratory risk", "length of stay", "ICU mortality", "ventilation duration", etc. Different types of outcomes are possible, e.g., binary outcomes (e.g., high hemodynamic risk or not), for which a likelihood of the outcome occurring may be determined; categorical outcomes (e.g., high/middle/low hemodynamic risk), for which a probability distribution among the possible outcomes may be determined; or numerical outcomes (e.g., length of stay, ventilation duration), for which an expected value or a probability distribution among expected values may be determined. For example, outcomes 538, 539 indicate that for patients for which the action recommended by model **M1** was followed, historically 50% of cases reached outcome 531 and 50% of cases reached outcome 532; for patients for which the action recommended by model **M2** was followed, 80% of patients had outcome 531 and 20% of patients had outcome 532. The user may have the option of selecting specific outcomes or to get results on all available outcome measures.

By using the interactive decision table, the user may assess the model(s) on a subpopulation of interest and quantify relevant patient outcomes based on different model decisions. This allows the user to model a what-if scenario where different actions chosen on the same cohort lead to different patient outcomes.

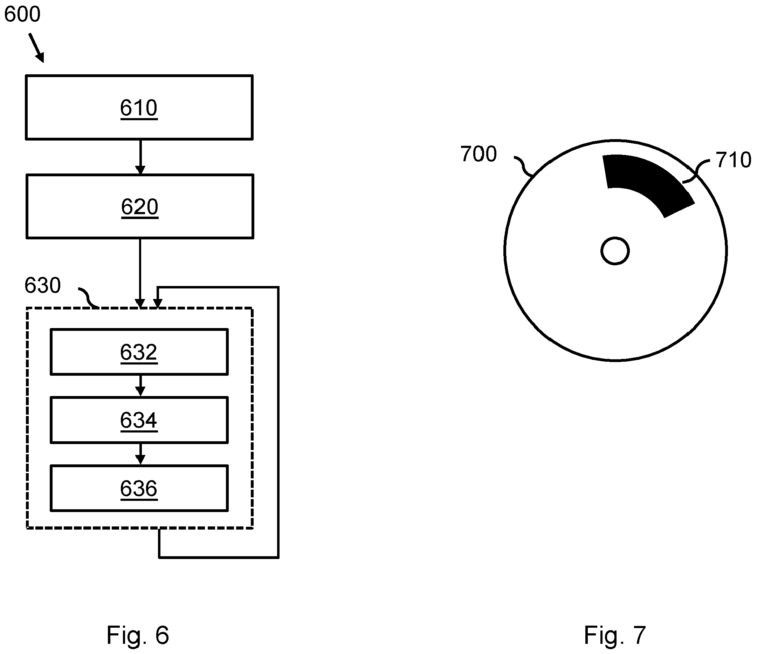

Fig. 6 shows a block-diagram of computer-implemented method 600 for use in intravenous fluid application to a patient. The method 600 may correspond to an operation of the system 200 of Fig. 2. However, this is not a limitation, in that the method 600 may also be performed using another system, apparatus or device.

The method 600 may comprise, in an operation titled "ACCESS MODEL", accessing 610 model data representing a trained fluid balance control model. The fluid balance control model may be configured to determine a target fluid balance based on a state of the patient. The state may comprise measurements of one or more physiological parameters of the patient.

The method 600 may comprise, in an operation titled "OBTAIN SENSOR DATA", obtaining 620 sensor data representing current measurements of the one or more physiological parameters.

The method 600 may comprise, in an operation titled, "MANAGE FLUID", performing 630 a management of the fluid application. The fluid management may comprise, in an operation titled "OBTAIN STATE", obtaining 632 the state of the patient, which may comprise obtaining the current measurements of the one or more physiological parameters via a sensor interface. The fluid management may comprise, in an operation titled "APPLY MODEL", applying 634 the fluid balance control model to the state of the patient to determine the target fluid balance. The fluid management may comprise, in an operation titled "OUTPUT BALANCE", outputting 636 the determined target fluid balance.

Further envisaged is a computer-implemented training method (not shown) of training a fluid balance control model for use as described herein, e.g., by the system of Fig. 2 and/or using the method of Fig. 6. This method may be combined with the method of Fig. 6, e.g., the model may be trained and subsequently applied. The training method may comprise accessing a training dataset of past fluid application instances and model data representing the fluid balance control model being trained. The training method may comprise training the model on the training dataset. The training method may comprise outputting trained model data representing the trained model.

It will be appreciated that, in general, the operations of method 600 of Fig. 6, and/or of the described training method, may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in Fig. 7, instructions for the computer, e.g., executable code, may be stored on a computer readable medium 700, e.g., in the form of a series 710 of machine-readable physical marks and/or as a series of elements having different electrical, e.g., magnetic, or optical properties or values. The medium 700 may be transitory or non-transitory. Examples of computer readable mediums include memory devices, optical storage devices, integrated circuits, servers, online software, etc. Fig. 7 shows an optical disc 700. Instead or in addition, the computer readable medium 700 may comprise model data 710 representing a trained fluid balance control model. The fluid balance control model may be configured to determine a target fluid balance based on a state of the patient. The state comprises measurements of one or more physiological parameters of the patient. The model may have been trained and/or may be for use as described herein.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

**Claims**

1. A system (200) for use in intravenous fluid application to a patient, comprising:

   - a data interface (220) for accessing model data (040) representing a trained fluid balance control model, wherein the fluid balance control model is configured to determine a target fluid balance based on a state of the patient, wherein the state comprises measurements of one or more physiological parameters of the patient;
   - a sensor interface (260) for obtaining sensor data representing current measurements of the one or more physiological parameters;
   - a processor subsystem (240) configured to perform a management of the fluid application by:
   - obtaining the state of the patient, wherein the obtaining comprises obtaining the current measurements of the one or more physiological parameters via the sensor interface;
   - applying the fluid balance control model to the state of the patient to determine the target fluid balance; and
   - outputting the determined target fluid balance.

2. The system (200) of claim 1, wherein the fluid balance control model has been trained on a training dataset of past fluid application instances, wherein a past fluid application instance comprises a past state of a patient, a past target fluid balance, and a reward value indicating a clinical outcome resulting from applying the past target fluid balance to the patient in the past state.

3. The system (200) of claim 2, wherein the clinical outcome indicates an organ system function, in particular, one or more of a lactate level, a hemodynamic stability, a renal function, and a respiratory function.

4. The system (200) of claim 2 or 3, wherein the clinical outcome incorporates a subgroup mortality rate determined by: assigning the patient to a subgroup based on a state of the patient resulting from applying the past target fluid balance, and obtaining a mortality rate of the assigned subgroup.

5. The system (200) of any one of claims 2-4, wherein the fluid balance control model has been trained using the reward value by reweighting the past fluid application instance based on the reward value such that the weight assigned to the past fluid therapy instance increases with its reward value.

6. The system (200) of any one of claims 2-4, wherein the processor subsystem is configured to apply the fluid balance control model by: evaluating a Q-function on the state and on respective target fluid balances to obtain respective Q-function values; and selecting the target fluid balance based on the respective Q-function values.

7. The system (200) of any preceding claim, further comprising an output interface (260) for providing fluid application parameters to an infusion pump, wherein the fluid application parameters indicate an amount of fluid and/or medication to be administered to the patient.

8. The system (200) of claim 7, further comprising a controller configured to receive the determined target fluid balance and to automatically control administration of fluid and/or medication according to the determined target fluid balance.

9. The system (200) of any preceding claim, further comprising a display output interface (280) for displaying the determined target fluid balance to a user.

10. The system (200) of claim 9, wherein the system is configured to display target fluid balances determined according to one or more trained fluid balance control models by superimposing the target fluid balances on a time series of measured fluid balances of the patient.

11. The system (200) of claim 9 or 10, wherein the system comprises a patient monitor (290) on which the determined target fluid balance is displayed.

12. The system (200) of any preceding claim, wherein the system is configured to obtain a selection of a fluid application objective, and to select the fluid balance control model from multiple respective fluid balance control models trained for respective fluid application objectives.

13. The system (200) of any preceding claim, wherein the system is configured to:

   - access a dataset of past fluid application instances;
   - via an input interface, obtain a selection by a user of one or more patient criteria;
   - filter the dataset according to the one or more patient criteria to obtain a subset of patients satisfying the one or more patient criteria;
   - apply the fluid balance control model to the subset of patients to determine a target fluid balance for the subset of patients, and output the target fluid balance for the subset of patients.

14. A computer-implemented method (600) for use in intravenous fluid application to a patient, comprising:

   - accessing (610) model data representing a trained fluid balance control model, wherein the fluid balance control model is configured to determine a target fluid balance based on a state of the patient, wherein the state comprises measurements of one or more physiological parameters of the patient;
   - obtaining (620) sensor data representing current measurements of the one or more physiological parameters;
   - performing (630) a management of the fluid application by:
   - obtaining (632) the state of the patient, comprising obtaining the current measurements of the one or more physiological parameters via a sensor interface;
   - applying (634) the fluid balance control model to the state of the patient to determine the target fluid balance; and
   - outputting (636) the determined target fluid balance.

15. A transitory or non-transitory computer-readable medium (700) comprising data (710) representing

   - instructions which, when executed by a processor system, cause the processor system to perform the computer-implemented method according to claim 14; and/or

- model data representing a trained fluid balance control model, wherein the fluid balance control model is configured to determine a target fluid balance based on a state of the patient, wherein the state comprises measurements of one or more physiological parameters of the patient.

Fig. 2

Fig. 1

EP 4 181 149 A1

| | 310 | | | 320 | |
|---|---|---|---|---|---|

| 331 | 341 | 351 | | |
|---|---|---|---|---|

| S1 | A1 | R1 | | |
|---|---|---|---|---|
| | S2 | A2 | R2 | |

| | 332 | 342 | 352 |
|---|---|---|---|

...

**Fig. 3a**

St — 333

FBCM — 360

Pars — 370

AQ1 (-1250,-750) — 381

AQ2 (-750,-250) — 382

...

AQ5 (+750,+1250) — 383

At — 343

**Fig. 3b**

Fig. 4

EP 4 181 149 A1

| Cond. | |
|---|---|
| ..... | **+** — 518 |
| ..... | |
| ..... | **+** — 519 |
| ..... | |

— 510

| Act. | |
|---|---|
| FB (-1250,-750) | **M1** — 528 |
| ..... | |
| FB (+750,+1250) | **M2** — 529 |

— 520

| Outc. | |
|---|---|
| Out1 | **M1**: 50%, **M2**: 80% — 538 |
| Out2 | **M1**: 50%, **M2**: 20% — 539 |
| ..... | |

530

Fig. 5

600

610

620

630

632

634

636

Fig. 6

EP 4 181 149 A1

700    710

Fig. 7

Europäisches
Patentamt

European
Patent Office

Office européen
des brevets

# EUROPEAN SEARCH REPORT

Application Number

EP 21 21 6788

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOMOROWSKI MATTHIEU ET AL: "The Artificial Intelligence Clinician learns optimal treatment strategies for sepsis in intensive care", NATURE MEDICINE, NATURE PUBLISHING GROUP US, NEW YORK, vol. 24, no. 11, 22 October 2018 (2018-10-22), pages 1716-1720, XP036901011, ISSN: 1078-8956, DOI: 10.1038/S41591-018-0213-5 [retrieved on 2018-10-22] * the whole document * | 1-15 | INV. G16H20/17 |
| X | CHRISTINA X JI ET AL: "Trajectory Inspection: A Method for Iterative Clinician-Driven Design of Reinforcement Learning Studies", ARXIV.ORG, CORNELL UNIVERSITY LIBRARY, 201 OLIN LIBRARY CORNELL UNIVERSITY ITHACA, NY 14853, 21 December 2020 (2020-12-21), XP081842396, * the whole document * | 1-15 | |
| X | JOSEPH FUTOMA ET AL: "Identifying Distinct, Effective Treatments for Acute Hypotension with SODA-RL: Safely Optimized Diverse Accurate Reinforcement Learning", ARXIV.ORG, CORNELL UNIVERSITY LIBRARY, 201 OLIN LIBRARY CORNELL UNIVERSITY ITHACA, NY 14853, 9 January 2020 (2020-01-09), XP081576006, * the whole document * | 1-15 | **TECHNICAL FIELDS SEARCHED (IPC)** G16H |
| A | US 2014/142539 A1 (SALINAS JOSE [US] ET AL) 22 May 2014 (2014-05-22) * paragraphs [0050] - [0121] * | 1-15 | |

-/--

The present search report has been drawn up for all claims

| Place of search | Date of completion of the search | Examiner |
|---|---|---|
| Munich | 7 June 2022 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 21 21 6788**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2016/279329 A1 (FAISAL ALDO [GB] ET AL) 29 September 2016 (2016-09-29) * paragraphs [0048] – [0085] * ----- | 1-15 | |
| A | CHAO YU ET AL: "Reinforcement Learning in Healthcare: A Survey", ARXIV.ORG, CORNELL UNIVERSITY LIBRARY, 201 OLIN LIBRARY CORNELL UNIVERSITY ITHACA, NY 14853, 22 August 2019 (2019-08-22), XP081641393, * the whole document * ----- | 1-15 | |

TECHNICAL FIELDS
SEARCHED       (IPC)

The present search report has been drawn up for all claims

| Place of search | Date of completion of the search | Examiner |
|---|---|---|
| Munich | 7 June 2022 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

......................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 21 6788

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2022

| Patent document cited in search report | | Publication date | Patent family member(s) | | Publication date |
|---|---|---|---|---|---|
| US 2014142539 | A1 | 22-05-2014 | US | 7857803 B1 | 28-12-2010 |
| | | | US | 7879020 B1 | 01-02-2011 |
| | | | US | 2011230824 A1 | 22-09-2011 |
| | | | US | 2012101473 A1 | 26-04-2012 |
| | | | US | 2012283631 A1 | 08-11-2012 |
| | | | US | 2014142539 A1 | 22-05-2014 |
| | | | US | 2014155818 A1 | 05-06-2014 |
| US 2016279329 | A1 | 29-09-2016 | EP | 3066599 A1 | 14-09-2016 |
| | | | US | 2016279329 A1 | 29-09-2016 |
| | | | WO | 2015067956 A1 | 14-05-2015 |

EPO FORM P0459

For more details about this annex : see Official Journal of the European Patent Office, No. 12/82